# FASCICULE DE BREVET EUROPEEN

(11) **EP 3 203 949 B1**
(45) Date de publication et mention de la délivrance du brevet: **17.03.2021**
(21) Numéro de dépôt: 15788339.8
(22) Date de dépôt: 07.10.2015
(51) Int. Cl.: A61F 9/008, B23K 26/06, B23K 26/067

(54) **DISPOSITIF POUR LA DECOUPE D'UNE CORNEE OU D'UN CRISTALLIN**
VORRICHTUNG ZUM SCHNEIDEN EINER HORNHAUT ODER AUGENLINSE
DEVICE FOR CUTTING A CORNEA OR A CRYSTALLINE LENS

(30) Priorité: 08.10.2014 FR 1459624
(43) Date de publication de la demande: 16.08.2017
(73) Titulaire: Université Jean Monnet Saint Etienne, 42000 Saint Etienne (FR); Centre National de la Recherche Scientifique, 75794 Paris Cedex 16 (FR)
(72) Inventeur: BERNARD, Aurélien, 42100 Saint Etienne (FR); GAIN, Philippe, 69009 Lyon (FR); MAUCLAIR, Cyril, 42660 Planfoy (FR); THURET, Gilles, 42170 Saint Just Saint Rambert (FR)
(74) Mandataire: Be IP
(86) Numéro de dépôt international: PCT/EP2015/073179
(87) Numéro de publication internationale: WO 2016/055539

(56) Documents cités:
- WO-A1-01/37769
- WO-A1-02/094117
- DE-A1-102007 019 812
- US-A1- 2010 133 246
- US-A1- 2012 271 286

## Description

### DOMAINE TECHNIQUE

La présente invention concerne le domaine technique des opérations chirurgicales réalisées au laser femtoseconde, et plus particulièrement celui de la chirurgie ophtalmologique pour notamment des applications de découpes de cornées, ou de cristallins.

L'invention concerne un dispositif de découpe d'un tissu humain ou animal, telle qu'une cornée, ou un cristallin, au moyen d'un laser femtoseconde.

L'invention trouve une application avantageuse, mais non limitative, dans la découpe de greffons de cornées conservés dans des banques de cornées, et dans la découpe cornéenne directement sur le patient pour des opérations de greffes de cornées, telles que trépanations verticales de différents profils, ou découpes lamellaires parallèles en surface.

Par laser femtoseconde, on entend une source lumineuse, apte à émettre un faisceau L.A.S.E.R. sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

### ART ANTERIEUR

Il est connu de l'état de la technique de réaliser des opérations chirurgicales de l'œil au moyen d'un laser femtoseconde, telles que des opérations de découpes de cornées ou de cristallins.

Le laser femtoseconde est donc un instrument apte à réaliser une dissection du tissu cornéen par exemple, en focalisant un faisceau L.A.S.E.R. dans le stroma de la cornée, et en réalisant une succession de petites bulles de cavitation adjacentes, qui forme ensuite une ligne de découpe.

Plus précisément, lors de la focalisation du faisceau L.A.S.E.R. dans la cornée, un plasma est généré par ionisation non-linéaire lorsque l'intensité du laser dépasse une valeur seuil, nommée seuil de claquage optique. Une bulle de cavitation se forme alors, engendrant une disruption très localisée des tissus environnant. Ainsi, le volume réellement ablaté par le laser est très faible comparativement à la zone disruptée.

La zone découpée par le laser à chaque impulsion est très petite, de l'ordre du micron ou de la dizaine de micron selon la puissance et la focalisation du faisceau. Ainsi, une découpe lamellaire cornéenne ne peut être obtenue qu'en réalisant une série d'impacts contigus sur toute la surface de la zone à découper.

Le déplacement du faisceau peut alors être réalisé par un dispositif de balayage, composé de miroirs galvanométriques pilotables, et/ou de platines permettant le déplacement d'éléments optiques, tels que des miroirs ou des lentilles. Une autre solution, réservée à la découpe de greffons, consiste à déplacer, non pas le faisceau L.A.S.E.R., mais le greffon lui-même au moyen de platines de déplacement automatisées.

Ces opérations de déplacement du faisceau L.A.S.E.R, ou du greffon lui-même sont longues et fastidieuses. L'opération chirurgicale de découpe est donc lente et plus difficile étant donné le temps allongé pendant lequel le patient peut avoir des mouvements d'oeil.

En effet, à titre d'exemple, le temps moyen de découpe d'une lamelle de 8 mm de diamètre dans une cornée humaine par un laser femtoseconde de cadence 5 kHz, avec des impacts séparés de 2 µm, est d'une quarantaine de minutes environ.

Pour optimiser le temps de découpe, il est connu d'augmenter la fréquence du laser. Cependant, l'augmentation de la fréquence implique également une augmentation de la vitesse de déplacement du faisceau, au moyen de platines ou de scanners adaptés. Il est également connu d'augmenter l'espacement entre les impacts du laser sur le tissu à découper, mais généralement au détriment de la qualité de la découpe.

La plupart des lasers femtosecondes pour la découpe cornéenne utilisent ainsi de hautes fréquences de travail, notamment supérieures à 100kHz, associées à des systèmes de déplacement du faisceau combinant des scanners et des platines de déplacement, ce qui grève le coût total de l'installation, et donc de l'opération chirurgicale facturée.

Pour remédier à ce problème de rapidité de la découpe L.A.S.E.R., il est aussi connu d'utiliser des miroirs galvanométriques pour augmenter la cadence, la vitesse, et le trajet de déflection du faisceau L.A.S.E.R..

Cependant, cette technique ne donne pas entière satisfaction en termes de résultats. La vitesse de la découpe peut être augmentée davantage.

Une autre solution pour diminuer le temps de découpe consiste à générer plusieurs bulles de cavitation simultanément. Les documents US 2010/0133246, EP 1 279 386 et DE 10 2007 019 812 décrivent des dispositifs de découpe basés sur la technique de subdivision d'un faisceau L.A.S.E.R. primaire unique en une pluralité de faisceaux L.A.S.E.R. secondaires. Ces dispositifs comprennent généralement un système optique - tel qu'un (ou plusieurs) séparateur(s) de faisceau - pour produire des faisceaux L.A.S.E.R. secondaires permettant de générer chacun une bulle de cavitation respective.

Le fait de générer simultanément « *n* » bulles de cavitation permet de diminuer la durée totale de la découpe d'un facteur « *n ».*

Toutefois, un inconvénient majeur de ces dispositifs est qu'il est très difficile d'homogénéiser l'énergie contenue dans chacun des faisceaux L.A.S.E.R. secondaires. En effet, il est nécessaire de modifier des éléments du système optique (par exemple modification de la position ou de l'orientation d'un séparateur de faisceau, suppression/remplacement/ajout d'une lentille, etc.) pour « régler » le système optique de sorte à générer des faisceaux L.A.S.E.R. secondaires homogènes. Ceci empêche d'uniformiser les dimensions des bulles de cavitation obtenues à partir de ces faisceaux L.A.S.E.R. secondaires et de maîtriser la position des différentes bulles de cavitation les unes par rapport aux autres.

Par ailleurs, la technique de subdivision induit une augmentation du diamètre de la pluralité de faisceaux L.A.S.E.R. secondaires par rapport au diamètre du faisceau L.A.S.E.R. primaire unique produit par le laser femtoseconde. En effet, les faisceaux L.A.S.E.R. secondaires correspondent à des « portions » du faisceau L.A.S.E.R. primaire unique séparées spatialement. Du fait de la distance non-nulle entre les différents faisceaux L.A.S.E.R. secondaires, le diamètre de l'ensemble que forment la pluralité de faisceaux L.A.S.E.R. secondaires est supérieur au diamètre du faisceau L.A.S.E.R. primaire.

Cette augmentation de diamètre peut être un inconvénient, notamment dans le cas où le dispositif de découpe comprend un système de balayage - tel qu'un scanner optique - pour déplacer la pluralité de faisceaux L.A.S.E.R. secondaires dans un plan de découpe. En effet, le diamètre d'entrée d'un système de balayage est généralement de l'ordre du diamètre du faisceau L.A.S.E.R. primaire unique de sorte que certains faisceaux secondaires ne pénètrent pas dans le système de balayage.

Un but de la présente invention est de proposer un dispositif de découpe permettant de pallier au moins l'un des inconvénients précités.

### EXPOSE DE L'INVENTION

L'invention est définie dans les revendications attenantes.

Ainsi donc, **l'invention** tend à proposer un dispositif de découpe d'un tissu humain ou animal, telle qu'une cornée, ou un cristallin, qui permet de réaliser des opérations de découpes rapides et viables.

Un autre objectif de l'invention est de fournir un tel dispositif qui soit de conception simple et peu onéreuse.

Afin de résoudre les problèmes précités, il a été mis au point, un dispositif de découpe comprenant, d'une manière connue, un laser femtoseconde apte à émettre un faisceau L.A.S.E.R. sous la forme d'impulsions, et des moyens aptes à diriger et focaliser ledit faisceau sur le tissu pour sa découpe en tant que telle.

Conformément à l'invention, le dispositif comprend, en outre, des moyens de mise en forme pour moduler la phase du front d'onde du faisceau L.A.S.E.R., positionnés sur la trajectoire dudit faisceau, et des moyens de commande pour piloter les moyens de mise en forme en leur appliquant une consigne déterminée de sorte à moduler la répartition d'énergie du faisceau L.A.S.E.R. en au moins deux points d'impact distincts dans son plan focal, correspondant au plan de la découpe.

Les moyens de commande comprennent par exemple un/des ordinateur(s), un/des processeur(s), un/des microcontrôleur(s), un/des micro-ordinateur(s), un/des automate(s) programmable(s), un/des circuit(s) intégré(s) spécifique(s) d'application, d'autres circuits programmables, ou d'autres dispositifs qui incluent un ordinateur tels qu'une station de travail.

On entend, dans le cadre de la présente invention, par *« point d'impact »* une zone du faisceau L.A.S.E.R. comprise dans son plan focal dans laquelle l'intensité dudit faisceau L.A.S.E.R. est suffisante pour générer une bulle de cavitation dans un tissu.

Ainsi, l'invention permet de modifier le profil d'intensité du faisceau L.A.S.E.R. dans le plan de la découpe, d'une manière à pouvoir améliorer la qualité ou bien la vitesse de la découpe en fonction du profil choisi. Cette modification de profil d'intensité est obtenue par modulation de la phase du faisceau L.A.S.E.R..

Le but de la mise en forme est de moduler la répartition finale d'énergie dans le faisceau, afin par exemple d'optimiser une découpe laser.

La modulation optique de phase est réalisée au moyen d'un masque de phase. L'énergie du faisceau L.A.S.E.R. incident est conservée après modulation, et la mise en forme du faisceau est réalisée en agissant sur son front d'onde. La phase d'une onde électromagnétique représente la situation instantanée de l'amplitude d'une onde électromagnétique. La phase dépend aussi bien du temps que de l'espace. Dans le cas de la mise en forme spatiale d'un faisceau L.A.S.E.R., seules les variations dans l'espace de la phase sont considérées.

Le front d'onde est défini comme la surface des points d'un faisceau possédant une phase équivalente (i.e. la surface constituée des points dont les temps de parcours depuis la source ayant émis le faisceau sont égaux). La modification de la phase spatiale d'un faisceau passe donc par la modification de son front d'onde.

Selon l'invention, les moyens de mise en forme se présentent sous la forme d'un modulateur spatial de lumière à cristaux liquides.

Un tel modulateur, généralement connu sous le sigle SLM, de l'acronyme anglais *« Spatial Light Modulator »,* est constitué d'une couche de cristaux liquides à orientation contrôlée permettant de façonner d'une manière dynamique le front d'onde, et donc la phase du faisceau L.A.S.E.R..

Plus précisément, un SLM est un dispositif de modulation de lumière modulant la phase d'un faisceau électromagnétique à l'aide de cristaux liquides. Ce système exploite le principe d'anisotropie des cristaux liquides, c'est-à-dire la modification de l'indice des cristaux liquides, en fonction de leur orientation spatiale.

L'orientation des cristaux liquide peut être réalisée à l'aide d'un champ électrique. Ainsi, en modifiant localement l'indice des cristaux liquides, il est possible de modifier le front d'onde du faisceau laser. Ce système peut posséder une très forte résolution, compatible avec une mise en forme complexe de faisceaux.

Le masque de phase, c'est-à-dire la carte représentant comment la phase du faisceau doit être modifiée pour obtenir une répartition d'amplitude donnée, est généralement calculée par un algorithme itératif basé sur la transformée de Fourier, ou sur divers algorithmes d'optimisation, tels que des algorithmes génétiques, ou le recuit simulé.

Le SLM permet donc de façonner d'une manière dynamique le front d'onde du faisceau L.A.S.E.R. puisqu'il est paramétrable numériquement. Cette modulation permet la mise en forme du faisceau de découpe d'une manière dynamique et reconfigurable.

Selon une forme de réalisation particulière, l'énergie du faisceau L.A.S.E.R. est répartie de sorte à générer une pluralité de points d'impact L.A.S.E.R. dans un plan de focalisation du faisceau L.A.S.E.R..

On a déjà proposé des dispositifs utilisant un SLM (cf. US 2012/271286). Toutefois, dans ces dispositifs, le SLM est configuré pour corriger des aberrations du faisceau électromagnétique issu de la source de rayonnement (et non pour répartir l'énergie d'un faisceau L.A.S.E.R. en au moins deux points d'impact distincts dans son plan focal par modulation de la phase du front d'onde dudit faisceau L.A.S.E.R.).

Dans le cadre de la présente invention, la mise en forme permet, à partir d'un unique faisceau gaussien, de répartir son énergie en plusieurs spots, limités en taille et en nombre par la résolution des moyens de mise en forme, et par la puissance du faisceau. Le nombre de spots diminue ainsi d'autant de fois le temps nécessaire à l'opération de découpe chirurgicale. En plus d'une diminution du temps de découpe, la présente invention permet d'autres améliorations, telles qu'une meilleure qualité de surface après découpe ou une diminution de la mortalité endothéliale. Il est bien évident que la présente invention peut être combinée avec les techniques actuelles consistant au déplacement rapide du faisceau, et à une haute fréquence de découpe pour augmenter davantage la vitesse de découpe.

Ainsi, la modulation reconfigurable du front d'onde du L.A.S.E.R. femtoseconde permet de générer de multiples points de découpes simultanés formant un motif, chaque point du motif ayant chacun une position contrôlée sur une surface ou dans un volume de la cornée.

On entend, dans le cadre de la présente invention, par *« motif »* une pluralité de points d'impact L.A.S.E.R. générés simultanément dans un plan de focalisation d'un faisceau L.A.S.E.R. mis en forme - c'est-à-dire modulé en phase pour répartir son énergie en plusieurs spots distincts dans le plan de focalisation correspondant au plan de découpe du dispositif.

Cette technique permet de réaliser l'opération de découpe d'une manière plus rapide et plus efficace car elle met en œuvre plusieurs spots L.A.S.E.R. réalisant chacun une découpe et selon un profil contrôlé.

De préférence, la forme de chaque point est également modulable. Cette technique se couple parfaitement aux techniques existantes de scanners et/ou de déplacement de platines.

De préférence, les différents points du motif sont régulièrement espacés sur les deux dimensions du plan focal de manière à former un quadrillage de spots L.A.S.E.R.

Ainsi, un seul balayage du faisceau L.A.S.E.R. mis-en-forme pour générer simultanément une pluralité de points d'impact remplace une multitude de balayages d'un faisceau « *non mis en forme »* générant un point d'impact unique.

La divulgation vise également à fournir un procédé de découpe d'un tissu humain ou animal, telle qu'une cornée, ou un cristallin, au moyen d'un laser femtoseconde apte à émettre un faisceau L.A.S.E.R. sous la forme d'impulsions, focalisé sur le tissu pour sa découpe en tant que telle.

Selon la divulgation, et conformément à ce qui précède, le procédé consiste à :
- appliquer une consigne de modulation de phase à des moyens de mise en forme du faisceau L.A.S.E.R., positionnés sur la trajectoire dudit faisceau,
- moduler la phase du front d'onde du faisceau L.A.S.E.R. avec les moyens de mise en forme, la consigne de modulation étant calculée pour répartir l'énergie du faisceau L.A.S.E.R. en au moins deux points d'impacts dans son plan focal, correspondant au plan de la découpe.

Ainsi, le procédé permet une découpe rapide et viable.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées, dans lesquelles :
- la figure 1 est une représentation schématique d'un montage du dispositif de découpe selon l'invention ;
- la figure 2 est une représentation schématique d'une mise en forme possible du faisceau L.A.S.E.R. du dispositif de découpe selon l'invention ;
- la figue 3 est une représentation illustrant un masque de phase permettant d'obtenir la répartition d'énergie telle qu'elle apparait à la figure 2 ;
- la figure 4 est une représentation illustrant un greffon cornéen avant l'opération de découpe ;
- la figure 5 est une représentation similaire à celle de la figure 4, le greffon cornéen étant représenté après avoir été aplanie ;
- la figure 6 est une représentation similaire à celle de la figure 5, illustrant le greffon cornéen après une première découpe laser effectuée ;
- la figure 7 illustre une répartition d'intensité d'un faisceau L.A.S.E.R. dans son plan focal ;
- la figure 8 illustre la répartition d'intensité obtenue en modulant la phase du front d'un faisceau L.A.S.E.R grâce à un modulateur spatial de lumière

### EXPOSE DETAILLE DE L'INVENTION

L'invention concerne un dispositif de découpe (1) d'un tissu humain au moyen d'un laser femtoseconde (2). Dans la suite de la description, l'invention sera décrite, à titre d'exemple, pour la découpe d'une cornée (3) d'un œil humain ou animal.

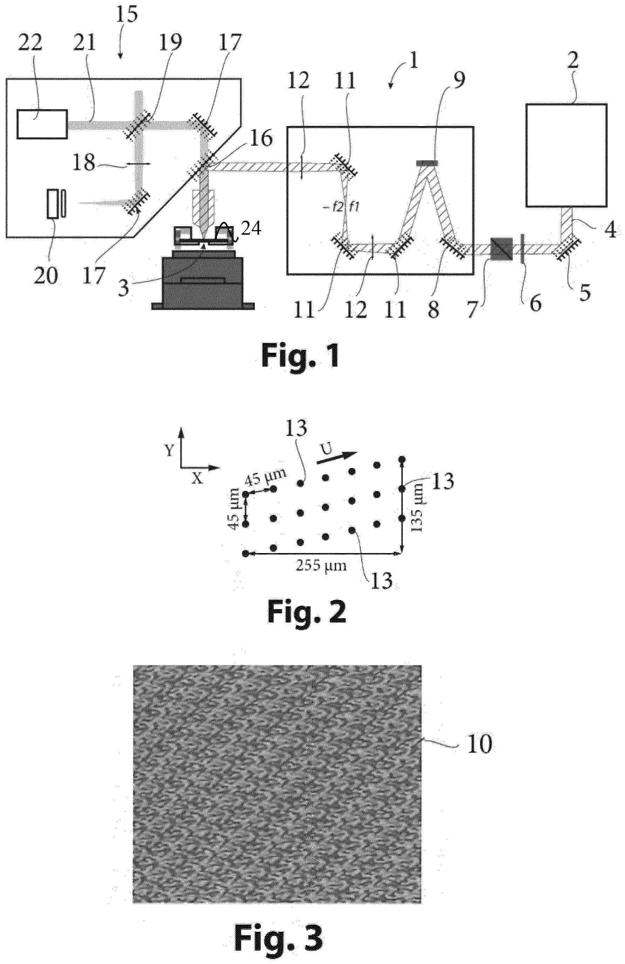

En référence à la figure 1 illustrant le montage d'un tel dispositif de découpe (1), celui-ci comprend un laser femtoseconde (2) apte à émettre un faisceau L.A.S.E.R. sous la forme d'impulsions. A titre d'exemple, le laser émet une lumière de 780 nm de longueur d'onde, sous la forme d'impulsions de 150 femtosecondes. Le laser possède une puissance de 2W et une fréquence de 5 kHz.

Le faisceau L.A.S.E.R. (4) émis par le laser (2) est dirigé et focalisé vers la cornée à découper par l'intermédiaire d'une pluralité d'éléments optiques. Plus précisément, un premier miroir (5) réfléchit le faisceau L.A.S.E.R. (4) issu directement du laser (2), et le renvoie vers une lame demi-onde (6) bien connue de l'état de la technique afin de créer un déphasage de 180°, c'est-à-dire un retard d'une moitié de longueur d'onde. L'onde sortante d'une telle lame (6) présente une polarisation symétrique de l'onde entrante par rapport à l'axe optique.

Le faisceau L.A.S.E.R. (4) issu de la lame demi-onde (6) traverse ensuite un cube polarisant (7) aussi connu de l'état de la technique, permettant de séparer la polarisation aléatoire du faisceau L.A.S.E.R. (4) en deux composantes de polarisation orthogonales et linéaires. L'une des composantes est réfléchie à 90°, tandis que l'autre composante est transmise. La composante de polarisation transmise est alors réfléchie sur un second miroir (8) jusqu'à des moyens de mise en forme (9) du faisceau L.A.S.E.R. (4).

Les moyens de mise en forme spatiale du faisceau L.A.S.E.R. (4) dans le plan focal permettent de faire varier la surface d'onde du faisceau L.A.S.E.R. (4) pour obtenir des points d'impact séparés les uns des autres dans le plan focal.

Plus précisément, les moyens de mise en forme permettent de moduler la phase du faisceau L.A.S.E.R. (4) issu du laser femtoseconde pour former des pics d'intensité dans le plan focal du faisceau, chaque pic d'intensité produisant un point d'impact respectif dans le plan focal correspondant au plan de découpe.

Les moyens de mise en forme sont, selon le mode de réalisation illustré, un modulateur spatial de lumière à cristaux liquides, connu sous le sigle SLM, de l'acronyme anglais « Spatial Light Modulator ».

Le SLM (9) permet de moduler la répartition finale d'énergie du faisceau L.A.S.E.R. (4), notamment dans le plan focal correspondant au plan de découpe de la cornée.

Plus précisément, le SLM est adapté pour modifier le profil spatial du front d'onde du faisceau L.A.S.E.R. (4) primaire issu du laser femtoseconde (4) pour distribuer l'énergie du faisceau L.A.S.E.R. (4) en différents spots de focalisation dans le plan de focalisation.

Le SLM (9) est un dispositif bien connu de l'état de la technique, il est constitué d'une couche de cristaux liquides à orientation contrôlée permettant de façonner d'une manière dynamique le front d'onde, et donc la phase du faisceau L.A.S.E.R. (4). La couche de cristaux liquides d'un SLM est organisée comme une grille (ou matrice) de pixels. L'épaisseur optique de chaque pixel est contrôlée électriquement par orientation des molécules de cristal liquide appartenant à la surface correspondant au pixel.

Le SLM (9) exploite le principe d'anisotropie des cristaux liquides, c'est-à-dire la modification de l'indice des cristaux liquides, en fonction de leur orientation spatiale. L'orientation des cristaux liquide peut être réalisée à l'aide d'un champ électrique. Ainsi, la modification de l'indice des cristaux liquides modifie le front d'onde du faisceau L.A.S.E.R. (4).

D'une manière connue, le SLM (9) met en œuvre un masque de phase (10), c'est-à-dire une carte déterminant comment la phase du faisceau (4) doit être modifiée pour obtenir une répartition d'amplitude donnée dans son plan de focalisation.

Le masque de phase est une image bidimensionnelle dont chaque point est associé à un pixel respectif du SLM. Ce masque de phase permet de piloter l'indice de chaque cristal liquide du SLM en convertissant la valeur associée à chaque point du masque - représentée en niveaux de gris compris entre 0 et 255 (donc du noir au blanc) - en une valeur de commande - représentée en une phase comprise entre 0 et 2π. Ainsi, le masque de phase est une consigne de modulation affichée sur le SLM pour entraîner en réflexion un déphasage spatial inégal du faisceau L.A.S.E.R. (4) illuminant le SLM. Bien entendu, l'homme du métier appréciera que la plage de niveau de gris peut varier en fonction du modèle de SLM utilisé. Par exemple dans certains cas, la plage de niveau de gris peut être comprise entre 0 et 220.

Le masque de phase (10) est généralement calculé par un algorithme itératif basé sur la transformée de Fourier, ou sur divers algorithmes d'optimisation, tels que des algorithmes génétiques, ou le recuit simulé. Différents masques de phase peuvent être appliqués aux SLM en fonction du nombre et de la position des points d'impact souhaités dans le plan focal du faisceau L.A.S.E.R. (4). Dans tous les cas, l'homme du métier sait calculer une valeur en chaque point du masque de phase pour distribuer l'énergie du faisceau L.A.S.E.R. (4) en différents spots de focalisation dans le plan de focal.

Le SLM (9) permet donc de façonner d'une manière dynamique le front d'onde du faisceau L.A.S.E.R. (4). Cette modulation permet la mise en forme du faisceau (4) de découpe d'une manière dynamique et reconfigurable.

Le SLM (9) permet, à partir d'un faisceau L.A.S.E.R. (4) gaussien générant un unique point d'impact, et au moyen du masque de phase (10) tel que représenté sur la figure 3, de répartir son énergie par modulation de phase de sorte à générer simultanément plusieurs points d'impact dans son plan de focalisation.

Ainsi l'invention propose de générer une pluralité de point d'impact à partir d'un unique faisceau L.A.S.E.R. mis en forme par modulation de phase (un seul faisceau en amont et en aval du SLM), contrairement aux dispositifs de US 2010/0133246, EP 1 279 386 et DE 10 2007 019 812 dans lesquels la pluralité de points d'impact L.A.S.E.R. est obtenue par subdivision d'un faisceau primaire en une pluralité de faisceau secondaires (un seul faisceau en amont d'un séparateur de faisceau et plusieurs faisceaux en aval du séparateur), chaque faisceau secondaire générant un point d'impact respectif.

Le faisceau L.A.S.E.R. (4) mis en forme par modulation de phase est ensuite dirigé vers une succession de miroirs (11) et de lentilles optiques (12), agencés pour diriger et focaliser ledit faisceau mis en forme par modulation de phase (4) sur la surface de la cornée (3) à découper. Une pluralité de spots L.A.S.E.R. (13) est alors focalisée sur la cornée (3), chaque spot (13) étant apte à réaliser une opération de découpe de la cornée (3).

En référence à la figure 2, les différents spots L.A.S.E.R. (13) obtenus sont, par exemple, régulièrement espacés sur les deux dimensions du plan focal du faisceau L.A.S.E.R. (4), de manière à former un quadrillage de spots L.A.S.E.R. (13). A titre d'exemple, la mise en forme du faisceau L.A.S.E.R. (4) par modulation de phase obtenue avec le masque de phase (10) peut permettre la formation d'un motif composé de trois lignes de 7 spots (13), espacés les uns des autres de 45 µm selon les deux dimensions dudit plan focal correspondant au plan de la découpe.

Le nombre de spots du motif diminue ainsi d'autant de fois le temps nécessaire à l'opération de découpe chirurgicale. En plus d'une diminution du temps de découpe de la cornée (3), la présente invention permet d'autres améliorations, telles qu'une meilleure qualité de surface après découpe ou une diminution de la mortalité endothéliale. Il est bien évident que la présente invention peut être combinée avec les techniques actuelles consistant au déplacement rapide du ou des faisceaux (4), et à une haute fréquence de découpe pour augmenter davantage la vitesse de découpe.

La modulation reconfigurable du front d'onde du L.A.S.E.R. femtoseconde permet de générer de multiples points de découpes simultanés ayant chacun une position contrôlée sur une surface ou dans un volume de la cornée (3).

Ainsi, il ressort de ce qui précède que l'invention permet donc de réaliser une opération de découpe chirurgicale d'une cornée, d'une manière rapide et efficace car elle met en œuvre plusieurs spots L.A.S.E.R. (13) réalisant chacun une découpe et selon un profil contrôlé.

Le SLM (9) peut également être configuré pour mettre en forme le front d'onde du faisceau L.A.S.E.R. (4) de toute autre manière. Par exemple, le spot L.A.S.E.R. obtenu pour réaliser la découpe de la cornée peut présenter une forme géométrique quelconque, autre que circulaire. Ceci peut présenter certains avantages en fonction de l'application considérée, comme une augmentation de la vitesse et/ou de la qualité de la découpe.

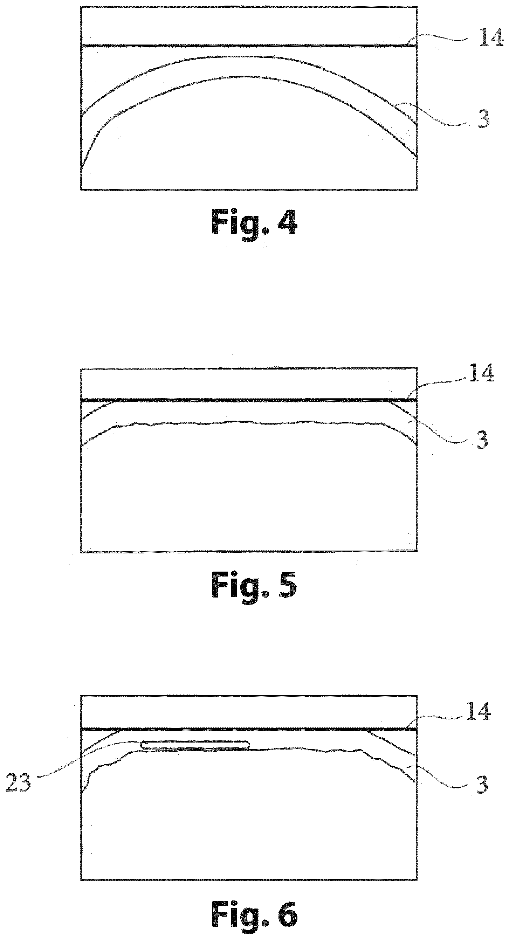

Avantageusement, et en référence aux figures 4 et 5, la surface de la cornée (3) à découper est aplanie par l'intermédiaire d'une lamelle d'aplanissement (14) bien connue de l'état de la technique. Cette lamelle (14) permet un aplanissement de la courbure cornéenne, simplifiant le trajet de découpe des spots L.A.S.E.R. (13) et améliorant ainsi la vitesse de découpe. La lamelle (14) sert également de référence pour le positionnement selon un axe Z des spots L.A.S.E.R. (13), c'est-à-dire selon un axe orthogonal au plan de la découpe. De cette manière, la lamelle d'aplanissement (14) permet une meilleure précision de découpe des greffons. Chaque spot (13) réalise un impact sur la cornée, vaporisant le tissu de ladite cornée de manière à former un point de découpe (23).

Enfin, afin de permettre un positionnement précis de la cornée (3) à découper, l'installation comprend un montage confocal de visualisation (15). Ce montage (15) permet l'obtention d'une précision de positionnement proche du micromètre de la cornée selon l'axe Z. Ce montage (15) comprend, en référence à la figure 1, un miroir dichroïque (16) et une lentille de focalisation (24) aptes à réfléchir, diriger et focaliser une partie de l'intensité du faisceau (4) mis en forme par modulation de phase, à savoir du faisceau (4) issu du SLM (9), vers la surface de la cornée (3) à découper. L'autre partie de l'intensité du faisceau (4) mis en forme est dirigée vers un agencement comprenant des miroirs (17), une lentille (18), et un second miroir dichroïque (19), agencés, d'une part, pour diriger une partie de l'intensité du faisceau (4) issu du miroir dichroïque (16) vers un capteur CCD (20) et, d'autre part, pour diriger un second faisceau L.A.S.E.R. (21), issu d'une seconde source de lumière (22) vers le miroir dichroïque (16) et la surface de la cornée (3) à découper. Ce montage (15) ne fait pas partie de l'invention et ne sera pas décrit plus en détail.

L'invention propose une méthode originale basée sur une modulation de la phase du front d'onde d'un faisceau L.A.S.E.R. pour redistribuer l'énergie dudit faisceau L.A.S.E.R. en une pluralité de points d'impact distincts dudit faisceau L.A.S.E.R.. On génère ainsi plusieurs points d'impacts à partir d'un seul faisceau L.A.S.E.R. modulé.

Ce phénomène peut être vu comme un phénomène d'interférences en deux dimensions. Chaque portion du faisceau L.A.S.E.R. initial issu de la source est retardée ou avancée par rapport au front d'onde initial afin que chacune de ces portions soit redirigée de façon à réaliser une interférence constructive en N points distincts dans le plan focal d'une lentille. Cette redistribution d'énergie en une pluralité de points d'impact n'a lieu que dans un seul plan (i.e. le plan de focalisation) et pas tout au long du chemin de propagation du faisceau L.A.S.E.R. modulé. Ainsi, l'observation du faisceau L.A.S.E.R. modulé avant ou après le plan de focalisation ne permet pas d'identifier une redistribution de l'énergie en une pluralité de points d'impact distincts, du fait de ce phénomène qu'on peut assimiler à des interférences constructives (qui n'ont lieu que dans un plan et pas tout au long de la propagation comme dans le cas de la séparation d'un faisceau L.A.S.E.R. initial en une pluralité de faisceaux L.A.S.E.R. secondaires).

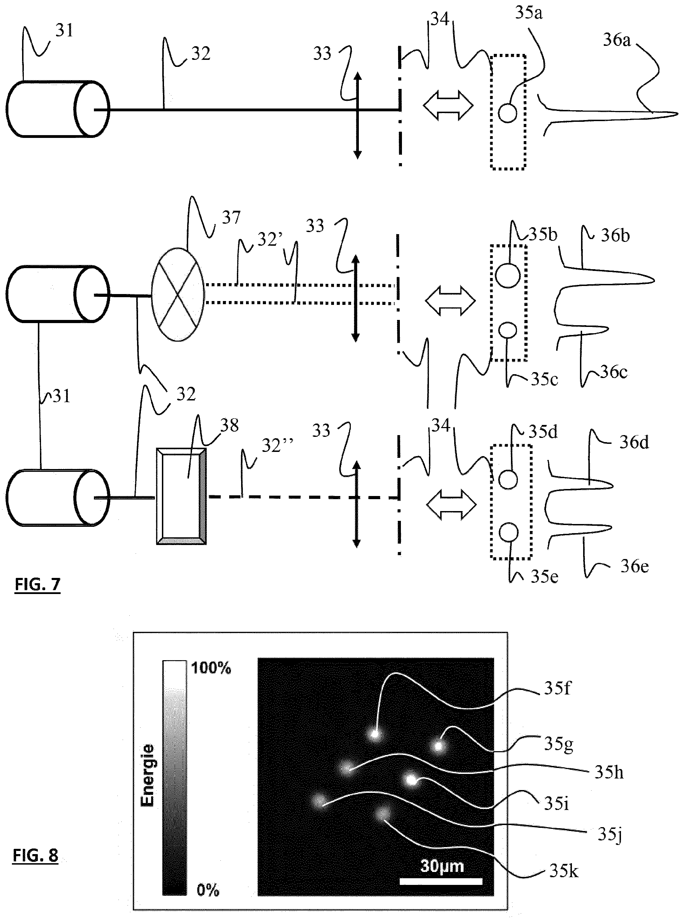

Pour mieux comprendre ce phénomène de modulation de phase du front d'onde, on a illustré schématiquement à la figure 7 des profils d'intensité 36a-36e obtenus pour trois exemples de montages optiques distincts.

Comme représenté à la figure 7, un faisceau L.A.S.E.R. 32 émis par une source laser 31 produit un pic d'intensité 36a de forme gaussienne en un point d'impact 35a dans un plan de focalisation 34.

L'insertion d'un séparateur de faisceau 37 entre la source 31 et le plan de focalisation 34 induit la génération d'une pluralité de faisceau L.A.S.E.R. secondaires 32', chaque faisceau L.A.S.E.R. secondaire 32' produisant un point d'impact 35b, 35c respectif dans le plan de focalisation 34 des faisceaux L.A.S.E.R. secondaires 32'.

Enfin, l'insertion entre la source 31 et le plan de focalisation 34 d'un SLM 38 programmé à l'aide d'un masque de phase formant consigne de modulation induit la modulation de la phase du front d'onde du faisceau L.A.S.E.R. 32 issu de la source 31. Le faisceau L.A.S.E.R. 32" dont la phase du front d'onde a été modulée permet d'induire la production de plusieurs pics d'intensité 36d, 36e séparés spatialement dans le plan focal 34 du faisceau L.A.S.E.R., chaque pic 36d, 36e correspondant à un point d'impact 35d, 35e respectif réalisant une découpe.

La méthode originale selon l'invention basée sur une modulation de la phase du front d'onde permet de générer plusieurs bulles de cavitation simultanées sans démultiplication du faisceau L.A.S.E.R. initial produit par la source L.A.S.E.R. femtoseconde, contrairement aux systèmes et procédés proposés dans l'art antérieur qui utilisent des dispositifs optiques de duplication de faisceau tels que des séparateurs de faisceau (cf. US 2010/0133246, EP 1 279 386 et DE 10 2007 019 812).

Pour mieux comprendre ce phénomène de modulation, considérons une onde se propageant à travers un système optique que l'on assimilera à une lentille mince de focale f. Les champs électriques objet *̅E̅*̅(*r̅,z*) et image *E̅(r̅*, *z*') situés respectivement dans les plans focaux objet et image d'une lentille sont liés par la relation suivante : *̅E̅*̅(*r̅'*,*z*') ∼ *TF*(*E̅(r̅,z* ))*.* D'autre part, le champ électrique d'une onde électromagnétique solution de l'équation de propagation peut s'exprimer sous la forme : *E*(*r̅, z*) = |*E*(*r̅,* z)|e^{*i*(*r̅,z*}), où (*r̅,z*) est appelée phase spatiale. Expérimentalement, on constate que l'influence de la phase spatiale dans le champ objet a une influence prépondérante sur la répartition d'amplitude dans le plan image. Il est alors possible, par un choix astucieux de la phase spatiale dans le champ objet, d'obtenir une répartition arbitraire de l'amplitude dans le champ image (dans le cas présent, au foyer de la lentille). Les mêmes considérations s'appliquent lorsque les champs objet et image ne coïncident pas avec les plans focaux. Il faut alors tenir compte de la propagation de l'onde dans le calcul de la phase (calcul non détaillé ici).

L'adressage numérique du SLM permet de faciliter sa programmation. Il est ainsi possible de régler finement le SLM pour obtenir des points d'impact 35d, 35e homogènes dans le plan de focalisation 34, ce qui n'est pas possible avec la technique de séparation de faisceau pour laquelle les dimensions et positions des points d'impacts 35b, 35c obtenus peuvent être très hétérogènes et pour lesquelles dans ce cas, il n'est pas possible de corriger ces défauts de manière dynamique, les techniques de séparation du faisceau étant basées sur des éléments optiques rigides.

Le réglage fin du SLM est réalisé en faisant varier le masque de phase utilisé pour le piloter.

Le réglage du SLM peut être mis en œuvre en plaçant un analyseur de faisceau (tel qu'une caméra CCD) dans le plan de focalisation et en projetant le faisceau L.A.S.E.R. modulé sur l'analyseur de faisceau. On fait alors varier les valeurs du masque de phase jusqu'à obtention de pics d'intensité de dimensions homogènes uniformément répartis. Une fois le masque de phase calculé précisément, celui-ci peut être utilisé dans tous les dispositifs de découpe fabriqués. Il est enregistré en tant que consigne de modulation dans les mémoires de moyens de commande des dispositifs pour piloter leur SLM respectif à l'aide dudit masque de phase. Ainsi une fois calculé, le masque de phase est figé et n'est pas modifié en fonction des propriétés (i.e. forme du front d'onde) du faisceau L.A.S.E.R. auquel SLM est associé.

En ce sens, le masque de phase est calculé indépendamment de la forme du front d'onde du faisceau L.A.S.E.R. avant modulation, contrairement au masque de phase des SLM utilisés pour corriger des aberrations comme proposé dans l'art antérieur.

A titre d'exemple, la modulation de phase a permis de réaliser expérimentalement une matrice de spots laser 35f-35k d'une uniformité telle que chaque spot 35f, 35g, 35h, 35i, 35j, 35k présente la même fluence crête à moins de 5% près, mesure réalisée à l'aide d'un capteur CCD, comme illustré à la figure 8.

La génération simultanée de plusieurs points d'impact par duplication de faisceau ne permet pas de maîtriser aussi facilement et précisément la position et les dimensions en section des différents faisceaux secondaires.

Ainsi, l'invention permet de disposer d'un outil de découpe efficace, puisque les impacts L.A.S.E.R. étant obtenus avec des spots d'énergie sensiblement égaux, les bulles de cavitation qui dilacèrent les tissus biologiques découpés seront de tailles sensiblement égales. Ceci permet d'améliorer la qualité du résultat obtenu, avec un plan de découpe homogène, dans lequel les ponts tissulaires résiduels ont tous sensiblement la même taille et qui permettent une dissection par le praticien d'une qualité acceptable au regard de l'importance de la qualité de l'état de surface du tissu découpé lorsqu'il s'agit par exemple d'une cornée. Les systèmes et procédés proposés dans l'art antérieur qui utilisent des dispositifs optiques de duplication de faisceau tels que des séparateurs de faisceau (cf. US 2010/0133246, EP 1 279 386 et DE 10 2007 019 812) ne permettent pas d'obtenir un plan de découpe homogène du fait de l'impossibilité de contrôler précisément l'emplacement de chaque faisceau et la répartition de l'énergie dans chaque faisceau, ce qui conduit à une découpe tissulaire non homogène, avec des ponts tissulaires de taille différente, et une dissection parfois facile, parfois difficile, ce qui ne permet pas de garantir un état de surface acceptable du tissu découpé.

Par ailleurs, à nombre de points d'impact identiques, le diamètre en section d'une pluralité de faisceaux dupliqués est supérieur au diamètre en section d'un faisceau L.A.S.E.R. modulé en phase selon l'invention. Ceci est dû au fait que les faisceaux dupliqués doivent être espacés d'une distance suffisante pour limiter les risques d'interférences.

Ainsi pour générer une pluralité de points d'impact, il sera plus facile d'associer un faisceau L.A.S.E.R. modulé en phase selon l'invention à un élément optique ayant une entrée de dimensions limitées plutôt qu'une pluralité de faisceau L.A.S.E.R. secondaires.

Par exemple, le faisceau L.A.S.E.R. modulé en phase selon l'invention est compatible avec l'utilisation d'un scanner optique de balayage composé d'un (ou plusieurs) miroir(s) optique(s) pivotant autour d'au moins deux axes.

L'intégration d'un tel scanner optique dans le dispositif de découpe selon l'invention permet de déplacer le motif de points d'impact (formé par le faisceau L.A.S.E.R. modulé en phase pour répartir l'énergie du faisceau L.A.S.E.R. en au moins deux points d'impact distincts) dans le plan de découpe en une pluralité de positions distinctes. Un tel système de déplacement peut être piloté par les moyens de commande du dispositif de découpe.

L'invention a été décrite pour des opérations de découpes d'une cornée (3) dans le domaine de la chirurgie ophtalmologique, mais il est évident qu'elle peut être utilisée pour d'autre type d'opération en chirurgie ophtalmologique sans sortir du cadre de l'invention. Par exemple, l'invention trouve une application dans la chirurgie réfractive cornéenne, tel que le traitement des amétropies, notamment myopie, hypermétropie, astigmatisme, dans le traitement de la perte d'accommodation, notamment la presbytie. L'invention trouve également une application dans le traitement de la cataracte avec incision de la cornée (3), découpe de la capsule antérieure du cristallin, et fragmentation du cristallin. Enfin, d'une manière plus générale, l'invention concerne toutes les applications cliniques ou expérimentales sur la cornée (3) ou le cristallin d'un œil humain ou animal.

D'une manière encore plus générale, l'invention concerne le domaine large de la chirurgie au L.A.S.E.R. et trouve une application avantageuse lorsqu'il s'agit de découper et plus particulièrement vaporiser des tissus mous humains ou animaux, à teneur en eau élevée.

## Revendications

1. Dispositif de découpe (1) d'un tissu humain ou animal, telle qu'une cornée (3), ou un cristallin, ledit dispositif (1) comprenant, un laser femtoseconde (2) apte à émettre un faisceau L.A.S.E.R. (4) sous la forme d'impulsions, et des moyens aptes à diriger et focaliser ledit faisceau L.A.S.E.R. (4) sur ou dans le tissu pour sa découpe en tant que telle, ledit dispositif (1) comprenant des moyens de mise en forme (9) positionnés sur la trajectoire dudit faisceau L.A.S.E.R. (4) pour moduler la phase du front d'onde du faisceau L.A.S.E.R. (4), selon une consigne de modulation calculée pour répartir l'énergie du faisceau L.A.S.E.R. en au moins deux points d'impact dans son plan focal, correspondant au plan de la découpe, ledit dispositif étant **caractérisé en ce que** lesdits moyens de mise en forme comprennent un modulateur spatial de lumière à cristaux liquides et **en ce que** le faisceau L.A.S.E.R. est un seul faisceau en amont et en aval dudit modulateur spatial de lumière.

2. Dispositif de découpe (1) selon la revendication 1, ***lequel*** comprend en outre des moyens de commande pour contrôler les moyens de mise en forme (9) en utilisant la consigne de modulation.

3. Dispositif de découpe (1) selon la revendication 1, ***dans lequel*** la consigne de modulation est une image bidimensionnelle à afficher sur le modulateur spatial de lumière pour entraîner en réflexion un déphasage spatial inégal du faisceau L.A.S.E.R. (4) induisant la répartition d'énergie du faisceau L.A.S.E.R. (4) en au moins deux points d'impact dans son plan focal.

4. Dispositif de découpe (1) selon l'une quelconque des revendications 1 à 3, ***dans lequel*** la consigne de modulation est une image bidimensionnelle à niveaux de gris composée d'une forme périodique répétée plusieurs fois dans l'image.

5. Dispositif de découpe (1) selon l'une quelconque des revendications 1 à 4, ***dans lequel*** la consigne de modulation est calculée indépendamment de la forme du front d'onde du faisceau L.A.S.E.R. (4) avant modulation.

6. Dispositif de découpe (1) selon l'une quelconques des revendications 1 à 5, ***dans lequel*** l'énergie du faisceau L.A.S.E.R. (4) est répartie dans le plan focal en une pluralité de points d'impact L.A.S.E.R. (4) distincts formant un motif, chaque point étant apte à réaliser une découpe du tissu.

7. Dispositif de découpe (1) selon la revendication 6, ***dans lequel*** les points d'impact L.A.S.E.R. du motif sont régulièrement espacés sur les deux dimensions du plan focal de manière à former un quadrillage de spots L.A.S.E.R. (13).

8. Dispositif de découpe (1) selon l'une quelconque des revendications 6 ou 7, ***lequel*** comprend en outre un système de miroir optique pivotant autour d'au moins deux axes pour déplacer le motif dans le plan de découpe en une pluralité de positions distinctes.

## Patentansprüche

1. Vorrichtung zum Schneiden (1) eines Gewebes eines Menschen oder Tieres wie eine Hornhaut (3) oder eine Augenlinse, wobei die Vorrichtung (1) einen Femtosekundenlaser (2) umfasst, der imstande ist, einen L.A.S.E.R.-Strahl (4) in Form von Impulsen zu senden, und Mittel, die imstande sind, den L.A.S.E.R.-Strahl (4) auf oder in das Gewebe für sein Schneiden an sich zu lenken und zu fokussieren, wobei die Vorrichtung (1) Formgebungsmittel (9) umfasst, die auf dem Weg des L.A.S.E.R.-Strahls (4) positioniert sind, um die Phase der Wellenfront des L.A.S.E.R.-Strahls (4) gemäß einem Modulationssollwert zu modulieren, der berechnet ist, um die Energie des L.A.S.E.R.-Strahls auf mindestens zwei Auftreffpunkte in seiner Fokalebene zu verteilen, die der Schneidebene entspricht, wobei die Vorrichtung **dadurch gekennzeichnet ist, dass** die Formgebungsmittel einen räumlichen Flüssigkeitskristall-Lichtmodulator umfassen und dass der L.A.S.E.R.-Strahl ein dem räumlichen Lichtmodulator vorgelagerter und nachgelagerter einziger Strahl ist.

2. Schneidvorrichtung (1) nach Anspruch 1, der ferner Steuermittel umfasst, um die Formgebungsmittel (9) durch Verwendung des Modulationssollwerts zu steuern.

3. Schneidvorrichtung (1) nach Anspruch 1, wobei der Modulationssollwert ein zweidimensionales, auf dem räumlichen Lichtmodulator anzuzeigendes Bild ist, um in Reflexion eine ungleiche räumliche Phasenverschiebung des L.A.S.E.R.-Strahls (4) zu bewirken, was die Verteilung der Energie des L.A.S.E.R.-Strahls (4) auf mindestens zwei Auftreffpunkte in seiner Fokalebene induziert.

4. Schneidvorrichtung (1) nach einem der Ansprüche 1 bis 3, wobei der Modulationssollwert ein zweidimensionales Graustufen-Bild ist, das aus einer im Bild mehrmals wiederholten periodischen Form zusammengesetzt ist.

5. Schneidvorrichtung (1) nach einem der Ansprüche 1 bis 4, wobei der Modulationssollwert unabhängig von der Form der Wellenfront des L.A.S.E.R.-Strahls (4) vor Modulation berechnet wird.

6. Schneidvorrichtung (1) nach einem der Ansprüche 1 bis 5, wobei die Energie des L.A.S.E.R.-Strahls (4) in der Fokalebene in einer Vielzahl unterschiedlicher L.A.S.E.R.-Auftreffpunkte (4), die ein Motiv bilden, verteilt ist, wobei jeder Punkt imstande ist, das Gewebe zu schneiden.

7. Schneidvorrichtung (1) nach Anspruch 6, wobei die L.A.S.E.R.-Auftreffpunkte des Motivs in den beiden Dimensionen der Fokalebene gleichmäßig beabstandet sind, so dass sie ein Gitter aus L.A.S.E.R.-Spots (13) bilden.

8. Schneidvorrichtung (1) nach einem der Ansprüche 6 oder 7, die ferner ein optisches Spiegelsystem umfasst, das um mindestens zwei Achsen dreht, um das Motiv in der Schneidebene in eine Vielzahl unterschiedlicher Positionen zu verlagern.

## Claims

1. A device for cutting (1) a human or animal tissue, such as a cornea (3) or a crystalline lens, said device (1) comprising a femtosecond laser (2) able to emit a L.A.S.E.R. beam (4) in the form of pulses, and means able to direct and focus said L.A.S.E.R. beam (4) on or in the tissue for its cutting as such, said device (1) comprising shaping means (9) positioned on the path of said L.A.S.E.R. beam (4) to modulate the phase of the wavefront of the L.A.S.E.R. beam (4), according to a modulation setpoint calculated to distribute the energy of the L.A.S.E.R. beam into at least two impact points in its focal plane, corresponding to the plane of the cutout, said device being **characterized in that** said shaping means comprise a liquid-crystal spatial light modulator and **in that** the L.A.S.E.R. beam is a single beam upstream and downstream of said spatial light modulator.

2. The cutting device (1) according to claim 1, which further comprises control means for monitoring the shaping means (9) by using the modulation setpoint.

3. The cutting device (1) according to claim 1, wherein the modulation setpoint is a two-dimensional image to be displayed on the spatial light modulator to cause in reflection an unequal spatial phase-shift of the L.A.S.E.R. beam (4) inducing the energy distribution of the L.A.S.E.R. beam (4) into at least two impact points in its focal plane.

4. The cutting device (1) according to any one of claims 1 to 3, wherein the modulation setpoint is a two-dimensional gray-scale image composed of a periodic shape repeated several times in the image.

5. The cutting device (1) according to any one of claims 1 to 4, wherein the modulation setpoint is calculated independently of the shape of the wavefront of the L.A.S.E.R. beam (4) before modulation.

6. The cutting device (1) according to any one of claims 1 to 5, wherein the energy of the L.A.S.E.R. beam (4) is distributed in the focal plane into a plurality of distinct L.A.S.E.R. impact points (4) forming a pattern, each point being able to carry out a cutting of the tissue.

7. The cutting device (1) according to claim 6, wherein the L.A.S.E.R. impact points of the pattern are evenly spaced on the two dimensions of the focal plane so as to form a grid of L.A.S.E.R. spots (13).

8. The cutting device (1) according to any one of claims 6 or 7, which further comprises an optical mirror system pivoting about at least two axes to move the pattern in the cutting plane in a plurality of distinct positions.
